# EUROPEAN PATENT SPECIFICATION

(11) **EP 2 007 897 B1**
(45) Date of publication and mention of the grant of the patent: **19.09.2012**
(21) Application number: 07732122.2
(22) Date of filing: 26.03.2007
(51) Int. Cl.: C12P 7/06, C12N 15/53, C12N 9/02, C12N 1/21, C12N 15/90

(54) **ENHANCEMENT OF MICROBIAL ETHANOL PRODUCTION**
VERBESSERUNG MIKROBIELLER ETHANOLPRODUKTION
AMÉLIORATION DE LA PRODUCTION MICROBIENNE D'ÉTHANOL

(30) Priority: 24.03.2006 GB 0605890
(43) Date of publication of application: 31.12.2008
(73) Proprietor: Bioconversion Technologies Limited, Frimley Green Camberley Surrey GU16 6LD (GB)
(72) Inventor: JAVED, Muhammad, Essex RM8 1YB (GB); BAGHAEI-YAZDI, Namdar, London W9 1SF (GB)
(74) Representative: Spencer, Matthew Peter
(86) International application number: PCT/GB2007/001060
(87) International publication number: WO 2007/110606

(56) References cited:
- WO-A-02/29030
- WO-A1-01/49865
- WO-A2-2007/110592
- US-A1- 2005 042 736
- LANE D.J. ET AL: 'PHYLOGENETIC ANALYSIS OF THE GENERA THIOBACILLUS AND THIOMICROSPIRA BY 5S RRNA SEQUENCES' JOURNAL OF BACTERIOLOGY, AMERICAN SOCIETY FOR MICROBIOLOGY, WASHINGTON, DC; US vol. 163, no. 1, 01 July 1985, pages 75 - 81, XP000608741 ISSN: 0021-9193
- NANBA H. ET AL: 'Purification and characterization of an alpha-haloketone-resistant formate dehydrogenase from Thiobacillus sp. strain KNK65MA, and cloning of the gene' BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN vol. 67, no. 10, 01 October 2003, pages 2145 - 2153, XP002356035 ISSN: 0916-8451

### SEQUENCE LISTING

<110> Bioconversion Technologies Limited
<120> Enhancement of microbial ethanol production
<130> P86806WO00
<150> GB 0605890.3
   <151> 2006-03-24
<160> 17
<170> PatentIn version 3.3
<210> 1
   <211> 1170
   <212> DNA
   <213> Artificial sequence
<220>
   <223> synthetic fdh gene
<400> 1
   atggcaaaag tactttgcgt tctttatgat gatccggtag atggctatcc gaagacgtat      60
   gcccgagatg atttaccgaa gatagatcac tatccaggag ggcaaacatt gccgacgccg     120
   aaagcaattg acttcacgcc tgggcaattg ttaggaagcg tatctggcga gctgggactt     180
   agaaagtatc ttgagtccaa tggacatacg ttagtggtaa ctagcgataa ggatgggcca     240
   gactcagtgt ttgaacggga gttagtggat gccgatgttg tcattagtca accgttctgg     300
   cctgcatatc ttacgccgga aagaatcgca aaagcgaaga acttgaaact agccctgaca     360
   gcaggaattg gaagcgatca tgtggatttg caaagcgcta ttgatcgcaa tgttaccgtg     420
   gcagaggtga catattgtaa ttctattagt gtagctgagc atgtggtaat gatgatttta     480
   tccttagtta gaaattactt gccgagccac gaatgggctc gtaaaggcgg gtggaatatt     540
   gcagattgcg tttctcatgc ttatgattta gaggcgatgc atgttggcac ggttgcggcg     600
   ggacgtatag gcttggcagt cttgcgtcga ctagcaccgt ttgacgttca tttacactat     660
   actgatcgac atcgtcttcc agagtccgtt gagaaagaac ttaacttaac ctggcatgca     720
   acgcgtgaag atatgtatcc ggtgtgtgac gtggtaacat tgaattgccc gttacatcct     780
   gaaactgaac acatgatcaa tgacgaaacg ttgaaactgt ttaaacgagg cgcttatatc     840
   gtaaacacag ccagagggaa actttgtgat cgggatgctg tagccagagc acttgagagc     900
   ggacgcttag ccgggtatgc aggcgacgtg tggtttccac aacctgcccc gaaagatcat     960
   ccgtggagaa cgatgccgta taatggaatg acgccacata tttcaggcac tacgttaaca    1020
   gcacaagcac gttatgcggc cggcacccgt gaaattcttg agtgcttctt cgaaggccgt    1080
   ccgatccgag atgaatattt gattgtacaa ggtggcgcat tagctgggac aggagcacat    1140
   agttatagca aaggcaatgc tacgggaggc                                     1170
<210> 2
   <211> 1558
   <212> DNA
   <213> artificial sequence
<220>
   <223> synthetic fdh gene including promoter and terminator
<400> 2
   gcctctagaa gggcaatctg aaaggaaggg aaaattcctt tcggattgtc cttttagtta 60
   tttttatggg gagtgaatat tatataggca ttacggaaat gataatggca gagttttttc 120
   atttattaga ctgcttgatg taattggatg tgatgataca aaaataatgt tgtgtaaaca 180
   aaatgttaac aaaaaagaca aatttcattc atagttgata cttgataaag attgtgaaat 240
   aatgcacaat atatcaatgt atgagcagtt tcacaaattc attttttgga aaggatgaca 300
   gacagcgatg gcaaaagtac tttgcgttct ttatgatgat ccggtagatg gctatccgaa 360
   gacgtatgcc cgagatgatt taccgaagat agatcactat ccaggagggc aaacattgcc 420
   gacgccgaaa gcaattgact tcacgcctgg gcaattgtta ggaagcgtat ctggcgagct 480
   gggacttaga aagtatcttg agtccaatgg acatacgtta gtggtaacta gcgataagga 540
   tgggccagac tcagtgtttg aacgggagtt agtggatgcc gatgttgtca ttagtcaacc 600
   gttctggcct gcatatctta cgccggaaag aatcgcaaaa gcgaagaact tgaaactagc 660
   cctgacagca ggaattggaa gcgatcatgt ggatttgcaa agcgctattg atcgcaatgt 720
   taccgtggca gaggtgacat attgtaattc tattagtgta gctgagcatg tggtaatgat 780
   gattttatcc ttagttagaa attacttgcc gagccacgaa tgggctcgta aaggcgggtg 840
   gaatattgca gattgcgttt ctcatgctta tgatttagag gcgatgcatg ttggcacggt 900
   tgcggcggga cgtataggct tggcagtctt gcgtcgacta gcaccgtttg acgttcattt 960
   acactatact gatcgacatc gtcttccaga gtccgttgag aaagaactta acttaacctg 1020
   gcatgcaacg cgtgaagata tgtatccggt gtgtgacgtg gtaacattga attgcccgtt 1080
   acatcctgaa actgaacaca tgatcaatga cgaaacgttg aaactgttta aacgaggcgc 1140
   ttatatcgta aacacagcca gagggaaact ttgtgatcgg gatgctgtag ccagagcact 1200
   tgagagcgga cgcttagccg ggtatgcagg cgacgtgtgg tttccacaac ctgccccgaa 1260
   agatcatccg tggagaacga tgccgtataa tggaatgacg ccacatattt caggcactac 1320
   gttaacagca caagcacgtt atgcggccgg cacccgtgaa attcttgagt gcttcttcga 1380
   aggccgtccg atccgagatg aatatttgat tgtacaaggt ggcgcattag ctgggacagg 1440
   agcacatagt tatagcaaag gcaatgctac gggaggcagc gaggaagcag ctaaatttaa 1500
   gaaagcggtt taacacagca ggggctgatc ggcccctgtt atgtttcatt ctagagcc 1558
<210> 3
   <211> 401
   <212> PRT
   <213> Pseudomonas sp. 101
<400> 3
   Met Ala Lys Val Leu Cys Val Leu Tyr Asp Asp Pro Val Asp Gly Tyr
   1               5                   10                  15
   Pro Lys Thr Tyr Ala Arg Asp Asp Leu Pro Lys Ile Asp His Tyr Pro
               20                  25                  30
   Gly Gly Gln Thr Leu Pro Thr Pro Lys Ala Ile Asp Phe Thr Pro Gly
           35                  40                  45
   Gln Leu Leu Gly Ser Val Ser Gly Glu Leu Gly Leu Arg Lys Tyr Leu
       50                  55                  60
   Glu Ser Asn Gly His Thr Leu Val Val Thr Ser Asp Lys Asp Gly Pro
   65                  70                  75                  80
   Asp Ser Val Phe Glu Arg Glu Leu Val Asp Ala Asp Val Val Ile Ser
                   85                  90                  95
   Gln Pro Phe Trp Pro Ala Tyr Leu Thr Pro Glu Arg Ile Ala Lys Ala
               100                 105                 110
   Lys Asn Leu Lys Leu Ala Leu Thr Ala Gly Ile Gly Ser Asp His Val
           115                 120                 125
   Asp Leu Gln Ser Ala Ile Asp Arg Asn Val Thr Val Ala Glu Val Thr
       130                 135                 140
   Tyr Cys Asn Ser Ile Ser Val Ala Glu His Val Val Met Met Ile Leu
   145                 150                 155                 160
   Ser Leu Val Arg Asn Tyr Leu Pro Ser His Glu Trp Ala Arg Lys Gly
                   165                 170                 175
   Gly Trp Asn Ile Ala Asp Cys Val Ser His Ala Tyr Asp Leu Glu Ala
               180                 185                 190
   Met His Val Gly Thr Val Ala Ala Gly Arg Ile Gly Leu Ala Val Leu
           195                 200                 205
   Arg Arg Leu Ala Pro Phe Asp Val His Leu His Tyr Thr Asp Arg His
       210                 215                 220
   Arg Leu Pro Glu Ser Val Glu Lys Glu Leu Asn Leu Thr Trp His Ala
   225                 230                 235                 240
   Thr Arg Glu Asp Met Tyr Pro Val Cys Asp Val Val Thr Leu Asn Cys
                   245                 250                 255
   Pro Leu His Pro Glu Thr Glu His Met Ile Asn Asp Glu Thr Leu Lys
               260                 265                 270
   Leu Phe Lys Arg Gly Ala Tyr Ile Val Asn Thr Ala Arg Gly Lys Leu
           275                 280                 285
   Cys Asp Arg Asp Ala Val Ala Arg Ala Leu Glu Ser Gly Arg Leu Ala
       290                 295                 300
   Gly Tyr Ala Gly Asp Val Trp Phe Pro Gln Pro Ala Pro Lys Asp His
   305                 310                 315                 320
   Pro Trp Arg Thr Met Pro Tyr Asn Gly Met Thr Pro His Ile Ser Gly
                   325                 330                 335
   Thr Thr Leu Thr Ala Gln Ala Arg Tyr Ala Ala Gly Thr Arg Glu Ile
               340                 345                 350
   Leu Glu Cys Phe Phe Glu Gly Arg Pro Ile Arg Asp Glu Tyr Leu Ile
           355                 360                 365
   Val Gln Gly Gly Ala Leu Ala Gly Thr Gly Ala His Ser Tyr Ser Lys
       370                 375                 380
   Gly Asn Ala Thr Gly Gly Ser Glu Glu Ala Ala Lys Phe Lys Lys Ala
   385                 390                 395                 400
   Val
<210> 4
   <211> 307
   <212> DNA
   <213> Bacillus stearothermophilus strain LLD-R
<400> 4
   gcctctagaa gggcaatctg aaaggaaggg aaaattcctt tcggattgtc cttttagtta     60
   tttttatggg gagtgaatat tatataggca ttacggaaat gataatggca gagttttttc     120
   atttattaga ctgcttgatg taattggatg tgatgataca aaaataatgt tgtgtaaaca    180
   aaatgttaac aaaaaagaca aatttcattc atagttgata cttgataaag attgtgaaat    240
   aatgcacaat atatcaatgt atgagcagtt tcacaaattc attttttgga aaggatgaca    300
   gacagcg                                                              307
<210> 5
   <211> 3006
   <212> DNA
   <213> Bacillus stearothermophilus strain LLD-R
<400> 5
   tgtaaattat cactttattt ccgcacaaaa aagactcttt tttgcacatt ccttcggaat     60
   atccctctcc ccctttccga aagaatgtgc taaatttttt gtgaattatt tcggaatggg    120
   acatgggtga tttccagagg ggggacgagg acgtgattcg gtttcatgac tttcaggtcg    180
   atgtccagac atatgcccag cggggaaaac aaaacgactt tccccttctt aagcggtgcc    240
   ctcattgcca ggcaaaacgc cctctttatc gccatgggta ctacgaacga aatgccgtga    300
   cgtcgcatca gtcttatcgc atttggatcg ctcggtatcg ctgcccggag tgcaggagga    360
   cggtggccgt gttgccttca tttcttctcc cttattttca gtatacgttg cccaccatat    420
   ggagagtggt gaaagaacgg ttgggcctga ctccgaaacg ggggatggag gaggctccac    480
   tccttcctac ggatgaaggg gttttatttt atgtcccgac gtttattccg aaatttgaac    540
   caccttcatt ggttttttgc ggagcgctgg agaaaaattg gtcctgccat cgcccaagcc     600
   gagagaacga gccctatggt ggatccagac gatggaggag atcggcctct ttttcgtcat     660
   ccaagagata tgggagcacc gatcgacgca tctttttgca cgtacattca gttcctgatt    720
   tacttatatc cccttatatg gaatcattta tagattccca aacctttcct ctcgacggtc    780
   gggggaatga tccgatagga tagagacagg atggaccgat aaggtcctag aatgggatga    840
   acgaaggagg agatcgaaat gaatgagtcg atgagacagg agatcgcttt atttcggtat    900
   ggattgatcg ctccattggt gaatggacaa gtcgatccaa aacgtacttg aaggaagtag    960
   cggaacggat ccatcaagtt ccccaccatg gagagaaacg catcgccgcc aaaacgatcc   1020
   tcgactggtg cacgcagtac aaaaaagggg gctttgaggc gctgaagccg aaacgacggt   1080
   cggaccgtgg ccattcccgg aggctgtcac ctgaagaaga ggatcacatt ttagccctga   1140
   gaaaaaaaca cccccacatg cccgtgacgg tgttttacca acaccttatc gagcaggggg   1200
   aaatccaatc catctcttat ttcactatat accgactttt aaaaaaatac aacctcgtgg   1260
   ggaaagaaat tttaccgatt cctgaacgaa aacgattcgc gtacgatcag gtcaatgagc   1320
   tctggcaagg tgatttgtcc catggcccgt tgattcgcgt gaatggcaaa acgcaaaaaa   1380
   cgtttttgat tgcctatatc gatgactgct cgcgggtcgt gccgtacgct cagtttttct   1440
   cttccgagaa atttgacggg ttgcggatcg taaccgcgcg gagttaggga tcaccttgat   1500
   ccatacccag ccgtacgatc cgcaaagcaa agggaaaatc gaacgatttt tccgcaccgt   1560
   acagacgcgg ttttacccgt tgctcgaaat gaattcaccg aagtcgctcg aagagctaaa   1620
   cgagcgattt tggaagtggt tggaggaaga ttaccatcga aaaccgcatg cctcgttgaa   1680
   cgggaagacg ccacatgaag tgtttcaatc gcaagtccat ttggtgtcgt tcgtcgagga   1740
   ttcggattgg ctcgactcga tctttttgaa acgcgaatac cgtaaagtga aggccgatgg   1800
   tacggtcacg ttgaacaagc agctgtatga agttccgccc cggttcatcg gacaatcgat   1860
   cgaactccgt tatgatgaac aaggcgtgta tgtgtacgaa gacggtcaac gggtcgccga   1920
   agcggtcctt gttcgcttcg aggacaatgc ctatgtgaaa cgccatcggt caccgtttgc   1980
   ggcggttccg gtagagggag gcgaaaacga tgtataaaac gttttattcc ctttcccgag   2040
   agccgttttc gaaggagacg aatccaccag aggcttatca aggggcctcg tatcaagagg   2100
   ccctcgccgc tttggactac gtgaaacgaa caagagggat cgggctattg atcggtgaac   2160
   caggggccgg caagacattc gcccttcggg cgtttaagga atccctgaat ccgtcactgt   2220
   atcacgtcgt ttattttcca ttgtcaacgg gaagcgtgat ggacttttat cgcggccttg   2280
   ccttcgggct cggggaagag ccgaaatacc gcaaggtcga cttgttttat caaatccaac   2340
   aagggatcga gcgcttgtat catgaacaac gggtaacgtc agtgttcatc ctcgatgaaa   2400
   tgcatttagc gaaggatgcc tttctgcagg atatcgcgat cctgttcaac tttcacatgg   2460
   actcaacaaa tccgtttgtc ttgattttgg cggggctgcc ccatttacag gcaaaactac   2520
   ggttgaatca acaccgtccg cttcaccaac gaatcatcat gcgataccag atggggcctc 2580
   ttgataagga agaagtggta ggatatatcg aacaccgcct gaaacaggcg ggggcgaaac 2640
   acccgatttt taccccagct gccttagaag cgatcgccct gcagtcgcag gggtggccgc 2700
   ggatcatcaa caacctcgcc accacttgcc tgttatacgg cgctcaatta aaaaaacata 2760
   tgattgacga agacattgtg cgtatggcag ccgaagaaat ggggtactga cacagcaggg 2820
   gctgatcggc ccctgttatg tttcatcccg atccatcctc attctagtta atcatccgaa 2880
   ataatgtgca aatgttcgga aataatctgc aaaacctgga ataattcgca aagattttgc 2940
   acattatttc cgaatccgtc cgaaataatt tgaaaaaggg attctgaaat aatgtgctaa 3000
   tttaca 3006
<210> 6
   <211> 26
   <212> DNA
   <213> Artificial sequence
<220>
   <223> primer
<400> 6
   agtactgaaa tccggatttg atggcg 26
<210> 7
   <211> 24
   <212> DNA
   <213> Artificial sequence
<220>
   <223> primer
<400> 7
   agtactgcta aatttccaag tagc 24
<210> 8
   <211> 29
   <212> DNA
   <213> Artificial sequence
<220>
   <223> primer
<400> 8
   gaacgattct agatacagca agattccgc 29
<210> 9
   <211> 26
   <212> DNA
   <213> Artificial sequence
<220>
   <223> primer
<400> 9
   gtttgcgaat tcatagacgg acgcag 26
<210> 10
   <211> 28
   <212> DNA
   <213> Artificial sequence
<220>
   <223> primer
<400> 10
   acacagacgt cggcgatttg attcatac 28
<210> 11
   <211> 34
   <212> DNA
   <213> Artificial sequence
<220>
   <223> primer
<400> 11
   cgccatgacg tccatgataa ttactaatac tagg 34
<210> 12
   <211> 29
   <212> DNA
   <213> Artificial sequence
<220>
   <223> primer
<400> 12
   agggcaatct gaaaggaagg gaaaattcc 29
<210> 13
   <211> 27
   <212> DNA
   <213> Artificial sequence
<220>
   <223> primer
<400> 13
   tgcacagatc tccaccaaat cggcgtc 27
<210> 14
   <211> 29
   <212> DNA
   <213> Artificial sequence
<220>
   <223> primer
<400> 14
   ttgagcagat cttgatgcaa aacgataac 29
<210> 15
   <211> 25
   <212> DNA
   <213> Artificial sequence
<220>
   <223> primer
<400> 15
   taaagccgat gagcagcagt tgaag 25
<210> 16
   <211> 33
   <212> DNA
   <213> Artificial sequence
<220>
   <223> primer
<400> 16
   atattatcta gacattacgg aaatgataat ggc 33
<210> 17
   <211> 27
   <212> DNA
   <213> Artificial sequence
<220>
   <223> primer
<400> 17
   tcacaatcta gacaatcggc cataaac 27

## Description

### Field of the invention

This invention relates to fermentation procedures and microorganisms for use therein and in particular to the enhancement of microbial ethanol production. More specifically, the invention relates to enhanced ethanol production by thermophilic bacteria, such as Bacilli from mixed sugars derived from the hydrolysis of biomass. In particular, the invention envisages a novel pathway for ethanol production by cloning a gene which encodes an NAD-linked formate dehydrogenase enzyme into a thermophilic bacterium of the geuus Bacillus that possesses a functional gene which encodes a pyruvate-formate lyase enzyme complex but lacks lactate dehydrogenase activity.

### Background to the invention

Bioethanol is currently made from glucose, maltose or sucrose derived from cereal starch, sugar cane or sugar beet, which all have food value. Celluloses and hemicelluloses form a major part of agricultural by-products and could, in principle, be a major source of low-cost, renewable bio-ethanol. However, it is difficult and expensive to derive fermentable sugars from cellulose. In contrast, hemicelluloses are almost as abundant as cellulose and are easy to hydrolyse, but yield a mixture of mainly pentose sugars that yeasts cannot ferment.

For this reason, Hartley (see International Publication Number WO 88/09379) proposed production of ethanol by mutants of a thermophilic Bacillus, which very rapidly ferments all of the sugars derived from biomass, at temperatures up to 70°C. High ethanol yield is achieved only by stressed and moribund cells, however.

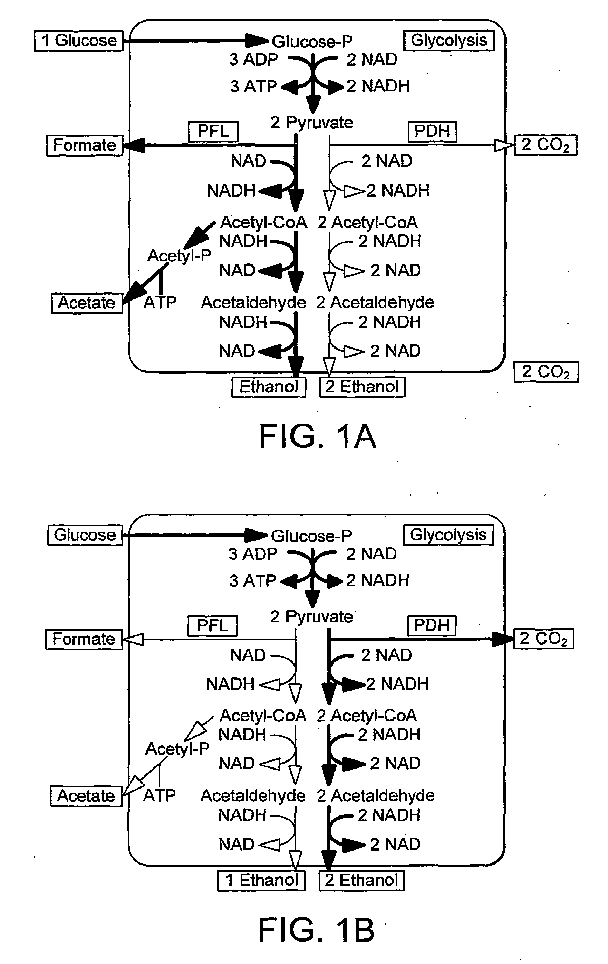

Many micro-organisms contain a pyruvate-formate lyase (PFL) pathway that converts pyruvate into acetyl CoA and formate (Figure 1A). Heterolactate fermentative microorganisms are one such class. These microorganisms first convert input sugars to pyruvate (generally by the EMP pathway of glycolysis), which then can take many routes to produce lactate, formate, acetate, ethanol and CO₂, in various proportions, depending on the growth conditions.

In fully aerobic cells, the pyruvate is normally metabolised to H₂O and CO₂ via the pyruvate dehydrogenase (PDH) pathway, tri-carboxylic acid cycle and the Electron Transport Chain. However, in many of these organisms, particularly thermophilic Bacilli, sugar uptake and glycolysis appear to be unregulated and lactate is a dominant product at high sugar concentrations, even under aerobic conditions. This suggests that the PDH flux has then become saturated, and that the excess pyruvate is diverted into an overflow lactate dehydrogenase pathway. This is not used for growth but produces heat which causes the ambient temperature to rise and kills mesophilic competitors, as can be seen when fresh grass is put on a compost heap.

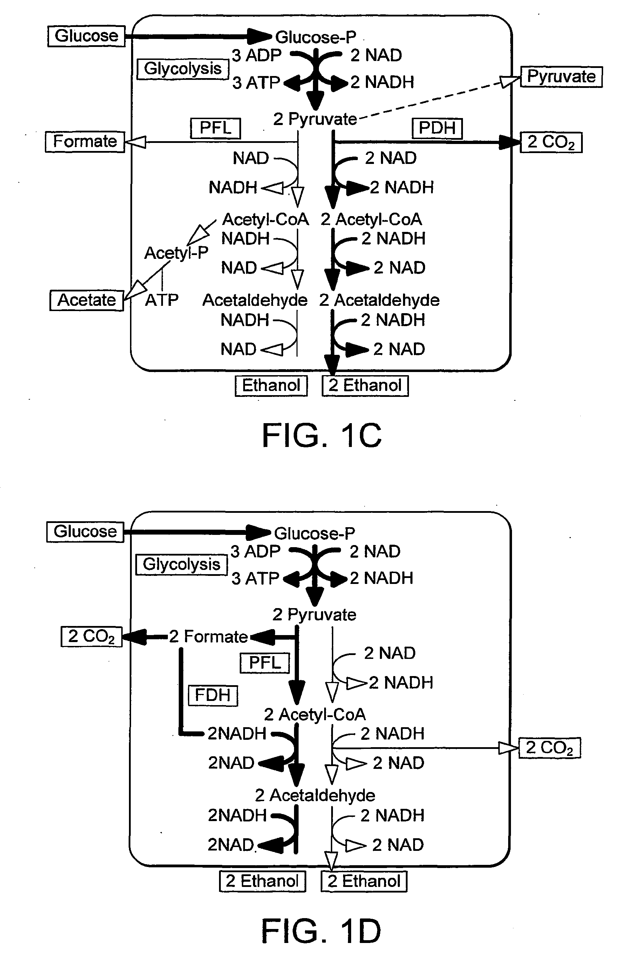

If the *ldh* gene (encoding lactate dehydrogenase) is inactivated, as described for example in WO 02/29030, lactate production stops and the excess pyruvate is diverted mainly into the growth-linked PFL pathway, (Figure 1A). However, at very high sugar concentrations and/or at acid pH, the PFL pathway flux declines and the excess pyruvate then overflows into an anaerobic PDH pathway, which yields only ethanol and CO₂ (Figure 1 B). Therefore the preferred conditions to obtain high ethanol yields are those that reduce flux through the PFL pathway and increase flux via the PDH pathway (Hartley, B.S. and Shama, G. Proc. Roy. Soc. Lond. 321, 555-568 (1987)). Unfortunately, under such conditions the cells experience metabolic stress, with reduced ATP production, and a potential imbalance in NAD/NADH and CoA/acetyl CoA ratios (Figure 1C).

Various fermentation protocols have been proposed to try to avoid or minimize this problem such as that of Hartley, B.S. as discussed above (see International Publication Number WO 88/09379) .

There are two classes of formate dehydrogenase. One (encoded by the *fdhF* gene) converts formate into CO₂ + H₂ and is typical of enterobacteriae such as *E. coli.* The other (encoded by the *fdh1* gene) converts formate + NAD into CO₂ + NADH₂ and is present in many facultative anaerobes. Berrios-Rivera et al (Metabolic Engineering 4, 217-219 (2002) replaced the *fdhF* gene in *E. coli* with a yeast *fdh1* gene and found that the reduced anaerobic products such as ethanol, lactate and succinate increased relative to oxidised products such as acetate. Building on this observation, San, K-Y. Berrios-Rivers, S.J. and Bennett, G.N. (see International Publication Number WO 2003/040690) proposed the introduction of an NAD-linked formate dehydrogenase gene as a general method to increase reducing power in cells involved in a broad range of bio-transformations. Subsequently San, K-Y. Bennett, G.N. and Sanchez, A. (US Patent Application US 2005/0042736 A1) proposed a specific application of this concept for production of succinate. These studies were carried out in *E. Coli,* an example of a mesophile where sugar uptake is regulated. The purpose of these experiments was to increase intracellular NADH levels so as to provide enhanced reducing power for various bio-transformations.

The yeast formate dehydrogenase recommended by Sen. et al (2004) is inactive at 60°C, which is the minimal growth temperature for the thermophilic bacteria potentially of use in bioethanol production. The most thermostable formate dehydrogenases so far described is the *Pseudomonas sp. 101* enzyme (A. Rojkova, A. Galkin, L. Kulakova, A. Serov, P. Savitsky, V. Fedorchuk, V. Tishkov FEBS Letters, Volume 445, Issue 1, Pages 183-188, 1999).

### Summary of the invention

The present invention attempts to solve the problems of producing high yields of ethanol from biomass. In particular, herein described for the first time is a novel metabolic pathway which allows thermophilic microorganisms, especially bacteria such as Bacillus to produce maximal ethanol yields. The invention is defined in the claims.

The invention relies upon thermophilic bacteria of the genus Bacillus which lack lactate dehydrogenase activity and thus require an alternative route for re-oxidation of excess NADH produced by glycolysis. This is provided by introduction into the thermophilic bacteria of the genus Bacillus of a gene encoding an NAD-linked formate dehydrogenase, such as an *fdh1* gene. In thermophilic microroganisms, and in contrast to mesophiles such as *E. coli,* sugar uptake is unregulated and this leads to accumulation of NADH in the presence of high levels of sugars. This eventually leads to a metabolic collapse and so-called "redox death" as shown schematically in figure 1C. Incorporation into the microorganism of a gene encoding an NAD-linked formate dehydrogenase helps to prevent cell death at high sugar concentrations by leading to a decrease in NADH levels and an increase in NAD levels. This is partly by restoring flux through the pyruvate dehydrogenase (PDH) pathway but most importantly, inclusion of a gene encoding an NAD-linked formate dehydrogenase creates a novel pyruvate formate lyase (PFL)-NAD-linked formate dehydrogenase (FDH) pathway for ethanol production. Figure 1D shows the potential for this PFL-FDH pathway to restore redox balance by converting all of the pyruvate produced by rapid glycolysis in the presence of high sugar levels to ethanol and CO₂, especially under neutral pH conditions. Importantly, the pathway operates under conditions that are optimal for cell growth, leading to rapid ethanol production and high yield, since the PFL pathway is the major growth-linked anaerobic pathway in thermophilic microorganisms.

Accordingly, in a first aspect the invention provides a thermophilic bacterium of the genus Bacillus, lacking lactate dehydrogenase (*ldh*) activity, characterised in that the thermophilic bacterium contains a gene encoding an NAD-linked formate dehydrogenase (*fdh*).

In one embodiment, the microorganism lacks lactate dehydrogenase activity by virtue of an appropriate gene deletion or other mutation which removes lactate dehydrogenase activity. Thus, preferably the ldh gene is deleted or otherwise rendered non-functional. Methods of gene knock-out and deletion are well known in the art and preferred examples are described in detail herein. Moreover, known strains of bacteria lacking lactate dehydrogenase activity (such as TN-T9 deposited under accession number NCIMB 41075 and TN-TK deposited under accession number NCIMB 41115) may be suitable for use in the present invention.

The thermophilic bacterium of the invention typically contains an active pyruvate formate lyase pathway. In particular, the microorganism preferably comprises a gene encoding a pyruvate formate lyase such as the *pf1* gene. The microorganisms of the invention typically also contain an active pyruvate dehydrogenase (PDH) pathway.

In a preferred embodiment, the gene encoding an NAD-linked formate dehydrogenase is integrated into the genome of the thermophilic bacterium. However, it is also possible for stable expression to be achieved without integration for example by introduction of a suitable plasmid.. One preferred method of integration is by recombination. The gene encoding an NAD-linked formate dehydrogenase may be operably linked to any suitable regulatory element to direct expression of the NAD-linked formate dehydrogenase. By "operably linked" is meant a functional linkage exists between the regulatory element and the gene encoding an NAD linked formate dehydrogenase. For example, the gene encoding an NAD-linked formate dehydrogenase may be linked to a suitable promoter which may be a constitutive or inducible promoter for example. "Promoter" is defined herein to include a region of DNA which is involved in the binding of RNA polymerase to initiate transcription.

Typically the promoter is a prokaryotic promoter and thus includes the appropriate -10 and -35 sequences, the consensus sequences of which are well defined in the art. The gene may also be operably linked to other appropriate regulatory sequences such as terminators for example. "Terminator" is defined as a nucleotide sequence which causes RNA polymerase to terminate transcription. In one embodiment, the gene encoding an NAD-linked formate dehydrogenase is expressed from its own promoter. In an alternative embodiment, the gene encoding an NAD-linked formate dehydrogenase is expressed from a promoter of the thermophilic bacterium (due to integration in an appropriate location in the genome). Constructions can also be envisaged where expression of the gene encoding an NAD-linked formate dehydrogenase is driven by a foreign promoter. This may be done to achieve maximal expression levels or inducible expression for example. As an example, phage promoters such as T7 may be utilised in conjunction with a suitable phage polymerase (which may be provided in a separate or the same DNA construct).

In a particularly preferred embodiment, the gene encoding an NAD-linked formate dehydrogenase is operably linked to the appropriate regulatory regions of a gene encoding a lactate dehydrogenase, in particular the upstream regulatory regions. The regulatory region preferably comprises the promoter of a gene encoding a lactate dehydrogenase. The promoter may be defined to include as a minimum functional unit the appropriate -10 and -35 sequences. Thus, according to one preferred embodiment of the invention the gene encoding an NAD-linked formate dehydrogenase is inserted into the lactate dehydrogenase gene of the thermophilic bacterium, thus inactivating the lactate dehydrogenase activity of the thermophilic bacterium. This embodiment is particularly preferred since both modifications required to produce a thermophilic bacterium of the invention are produced in the same step. Suitable constructs for achieving this are described in detail herein.

Ethanol production by thermophilic bacteria is advantageous since it can be carried out at high temperatures. Whilst thermophilic microorganisms have lower ethanol tolerance than yeasts, ethanol may be continuously and conveniently removed from the high temperature fermentation by membrane and/or mild vacuum evaporation. In optimal anaerobic growth conditions, Bacillus strain LLD-R grows very rapidly at 70°C almost exclusively by the PFL-pathway (Hartley and Shama, 1982). It can be envisaged that growth by the novel PFL-FDH pathway would be equally vigorous, but the maximum growth temperature would be limited by the thermostability of the NAD-linked formate dehydrogenase introduced into the thermophilic microorganism. The thermophilic bacterium of the invention may incorporate a gene encoding a thermostable NAD-linked formate dehydrogenase and/or a gene whose nucleotide sequence has been codon optimised to facilitate expression by a thermophilic microorganism. Production of such a thermostable NAD-linked formate dehydrogenase is described in detail herein. In a specific embodiment, the gene encoding an NAD-linked formate dehydrogenase comprises or consists of the nucleotide sequence set forth as SED ID NO: 1. In a further embodiment the thermophilic bacterium may of the invention incorporates a gene which is codon optimized for expression in Bacillus, encoding a thermostable NAD-linked formate dehydrogenase comprising or consisting of the nucleotide sequence set forth as SEQ ID NO:2. This sequence includes, in addition to the basic thermostable NAD-linked dehydrogenase sequence, promoter and terminator regions and also Xba1 sites to facilitate cloning of the gene into a suitable DNA construct.

The gene encoding an NAD-linked formate dehydrogenase can be the *fdh1* gene. The *fdh1* gene may be derived from any suitable source and is preferably codon optimised for expression in the relevant thermophilic microorganism.

The thermophilic bacterium of the invention may be produced by transformation with any of the DNA constructs of the invention as described in further detail herein. Accordingly, the discussion provided there applies mutatis mutandis to this embodiment of the invention.

The thermophilic microorganism of the invention is a thermophilic bacterium of the genus Bacillus and more preferably *Bacillus stearothermophilus*. The thermophilic microorganism of the invention may be derived from the known strain LLD-R or LLD-15 (of *Bacillus stearothermophilus*). The thermophilic bacterium described hexin can be *Geobacillus thermoglucosidasius.*

The fermentation processes facilitated by the present invention preferably utilise a synthetic NAD-linked formate dehydrogenase, designed to express a thermostable amino acid sequence due to use of the codon preferences of the appropriate thermophilic microorganism such as Bacillus strain LLD-R. The synthetic gene preferably contains engineered restriction sites to assist insertion into the lactate dehydrogenase gene. Thereby deletion of the LDH pathway and creation of the PFL-FDH pathway are achieved in a single operation. Accordingly, described herein is a thermostable NAD-linked formate dehydrogenase. Preferably, the thermostable NAD-linked formate dehydrogenase remains functional at or above a temperature of 60 °C. Preferably, the thermostable enzyme is encoded by a nucleotide sequence which has been codon optimised for expression in a thermophilic microorganism. The formate dehydrogenase may comprise, consist essentially of or consist of the amino acid sequence set forth as SEQ ID NO: 3.

A specific thermostable NAD-linked formate dehydrogenase has been designed based upon the amino acid sequence of the *Pseudomonas sp 101* formate dehydrogenase (SEQ ID NO:3) and through use of optimised codons for *Geobacillus thermoglucosidasius* as discussed in more detail in the detailed description below. The skilled person will appreciate that derivatives of this basic sequence will retain functionality. For example, conservative and semi-conservative substitutions may result in thermostable NAD-linked formate dehydrogenases which retain effective catalytic activity and thermostability such that they are useful in ethanol production using thermophilic microorganisms. Similarly, minor deletions and/or additions of amino acids may produce derivatives retaining appropriate functionality.

In a second aspect, the invention relates to a synthetic gene encoding a thermostable NAD-linked formate dehydrogenase. The gene comprises or consists of the nucleotide sequence set forth as SEQ ID NO:1. This sequence represents a novel fdh gene sequence in which the codons are optimised for production of a thermostable NAD-linked formate dehydrogenase. In a more specific embodiment, the gene encoding a thermostable NAD-linked formate dehydrogenase comprises or consists of the nucleotide sequence set forth as SEQ ID NO:2. This sequence incorporates the coding region for the thermostable NAD-linked formate dehydrogenase together with a suitable Bacillus promoter and rho-independent terminator. The sequence also incorporates suitable restriction sites to assist in cloning, in particular *Xba*1 sites. The skilled person will readily appreciate that minor modifications to the nucleotide sequence may be made without altering the functionality or thermostability of the resultant enzyme, for example through replacing optimized codons with other codons which are preferred in the translation systems of the appropriate thermophilic microorganism.

Also described herein is a DNA construct containing a gene encoding an NAD-linked formate dehydrogenase, in particular a thermostable NAD-linked formate dehydrogenase, wherein the gene is flanked by restriction sites to facilitate cloning of the gene into a suitable DNA construct, such as an expression vector or plasmid.

In a related aspect, described herein is a DNA construct comprising a regulatory sequence operably linked to a gene encoding a thermostable NAD-linked formate dehydrogenase. This DNA construct thus facilitates transformation of thermophilic bacteria of the genus Bacillus, in particular those lacking lactate dehydrogenase activity, in order to produce thermophilic microorganisms capable of efficient fermentation giving maximal ethanol yields. As aforementioned, the term "operably linked" as used herein refers to a functional linkage between the regulatory sequence and the gene encoding the NAD-linked formate dehydrogenase, such that the regulatory sequence is able to influence gene expression. For example, a preferred regulatory sequence is a promoter. As aforementioned, the gene encoding an NAD-linked formate dehydrogenase preferably may comprise, consist essentially of or consist of the nucleotide sequence set forth as SEQ ID NO:1. A preferred regulatory sequence is a promoter, although the DNA construct may additionally incorporate suitable terminator sequences. The promoter may comprise the nucleotide sequence set forth as SEQ ID NO:4. Other promoters, as discussed above, may be utilised for high levels and/or inducible expression.

Described herein is a DNA construct comprising a gene encoding an NAD-linked formate dehydrogenase and an insertion sequence, wherein the insertion sequence facilitates integration of the gene encoding an NAD-linked formate dehydrogenase into the genome of a thermophilic bacterium transformed with the DNA construct. By "insertion sequence" is meant a transposable DNA element which is capable of integration into the genome of the appropriate thermophilic microorganism. Insertion sequences may also be referred to as insertion sequence elements (IE) and may be naturally occurring. The insertion sequence may be derived from *Bacillus stearothermophilus* strain LLD-R or LLD-15.

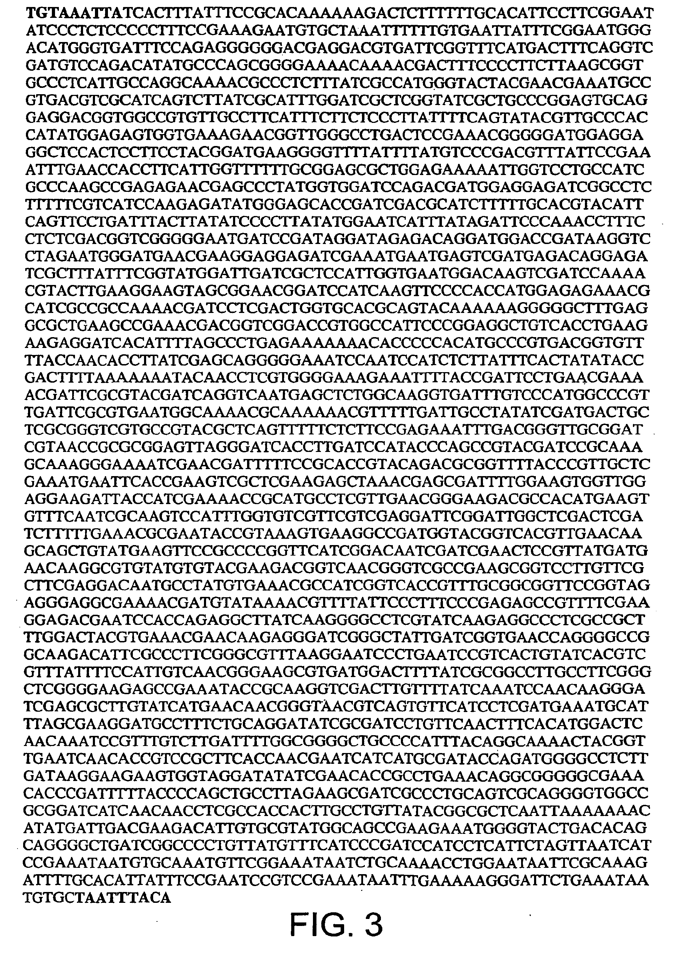

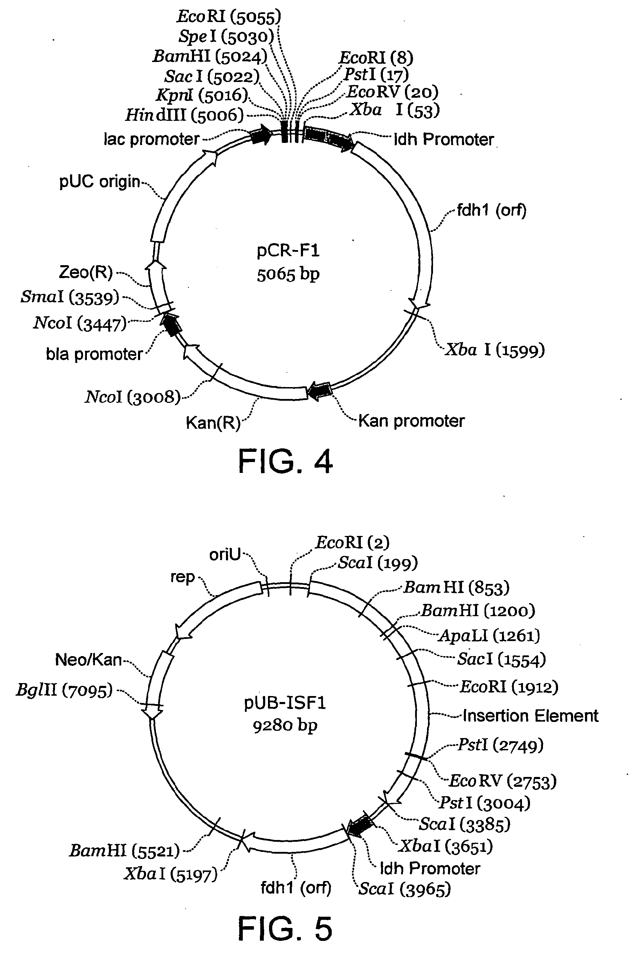

The insertion sequence may comprise, consist essentially of or consist of the nucleotide.sequence set forth as SEQ ID NO:5 (Figure 3). The preferred insertion sequence may be generated by amplification using primers comprising, consisting essentially of or consisting of the nucleotide sequence set forth as SEQ ID NO: 6 and 7. In this case, genomic DNA from the known *Bacillus stearothermophilus* strain LLD-15 may be used as the template. One particularly preferred DNA construct is plasmid pUB-ISF1 (as described in the experimental section below and in figure 5).

Described herein is a DNA construct comprising a (fdh) gene encoding an NAD-linked formate dehydrogenase operably linked to appropriate regulatory regions of a gene encoding a lactate dehydrogenase, in particular the upstream regulatory regions. The regulatory regions preferably comprise the promoter of a gene encoding a lactate dehydrogenase (ldh). The promoter may be defined to include as a minimum functional unit the appropriate -10 and -35 sequences to allow effective RNA polymerase binding. The lactate dehydrogenase gene promoter is suitable for driving high levels of expression in thermophilic bacteria such as Bacilli and also may advantageously be used as part of the cloning strategy to achieve both deletion of lactate dehydrogenase activity and introduction of NAD-linked formate dehydrogenase activity in the same step. The DNA construct may thus also be defined as comprising a gene encoding an NAD-linked formate dehydrogenase operably linked to a nucleic acid molecule which comprises the promoter of a gene encoding a lactate dehydrogenase (ldh).

The DNA construct preferably also contains part of the coding sequence of the host lactate dehydrogenase gene downstream of the gene encoding an NAD-linked formate dehydrogenase. This facilitates gene integration in a microorganism transformed with the DNA construct. By "at least part" is meant a portion of the gene of sufficient length to allow gene integration into the genome of a microorganism containing the lactate dehydrogenase gene by recombination (preferably by double cross-over). The part of the coding sequence preferably incorporates the end of the lactate dehydrogenase gene. At least 100, 200, 300, 400, 500, 600, 700 or 750 nucleotides of the lactate dehydrogenase gene can be incorporated downstream of the gene encoding an NAD-linked formate dehydrogenase. Thus, the DNA construct may comprise a gene encoding an NAD-linked formate dehydrogenase wherein the gene encoding an NAD-linked formate dehydrogenase is flanked by nucleotide sequence from a gene encoding a lactate dehydrogenase (derived from the thermophilic microorganism of interest). The flanking sequences are of sufficient length to allow integration of the gene encoding an NAD-linked formate dehydrogenase into the host gene encoding a lactate dehydrogenase to thereby introduce NAD-linked formate dehydrogenase activity and knock out lactate dehydrogenase activity in a single cloning step. Preferably, the gene encoding an NAD-linked formate dehydrogenase is flanked upstream by at least the promoter region of the gene encoding a lactate dehydrogenase, so that following integration by recombination the gene encoding an NAD-linked formate dehydrogenase is operably linked to the promoter. The downstream portion of the lactate dehydrogenase gene may be one obtainable by amplification of the ldh gene using primers comprising, consisting essentially of or consisting of the nucleotide sequence set forth as SEQ ID NO: 8 and 9, using the strain LLD-R as template. The upstream flanking region, which preferably incorporates the ldh promoter, preferably comprises at least 100, 200, 300, 400, 500, 600, 700 or 750 nucleotides of the appropriate ldh upstream regions to maximise efficiency of integration by recombination with the host genome. This upstream region may be dependent upon the sequence context of the ldh gene in the specific thermophilic microorganism of interest, as would be readily determined by a skilled person. Thus, the skilled person with knowledge of the ldh gene sequence would readily determine appropriate flanking regions to allow integration by recombination. For example, published genomic sequences may be studied, sequencing reactions carried out or flanking regions amplified by PCR using primers derived from the ldh gene sequence. Thus the fdh gene becomes interposed between two nucleotide sequences derived from the ldh gene such that the fdh gene replaces, in frame, at least part of the ldh gene.

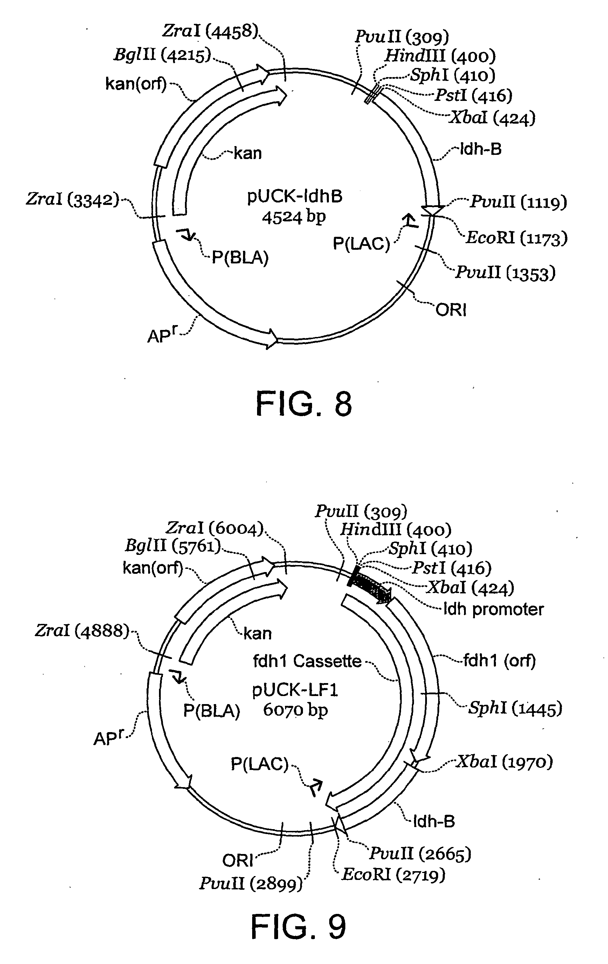

The DNA construct thus generally comprises a gene integration cassette in which the gene of interest (gene encoding an NAD-linked formate dehydrogenase) is inserted within the coding sequence (ORF) of a gene to be knocked out during integration (lactate dehydrogenase gene in this instance). Upon integration through recombination, expression of the gene of interest is in effect under the control of the knocked out gene. Such a construct may be of general applicability in the circumstance where one gene needs to be knocked out in favour of expression of a heterologous gene. In one preferred embodiment, the gene encoding an NAD-linked formate dehydrogenase encodes a thermostable NAD-linked formate dehydrogenase. The discussion of the thermostable NAD-linked formate dehydrogenases provided herein thus applies mutatis mutandis to this aspect of the invention. In particular, in one embodiment, the gene encoding a thermostable NAD-linked formate dehydrogenase comprises or consists of the nucleotide sequence set forth as SEQ ID NO:1 or 2. A particularly preferred DNA construct is plasmid pUCK-LF1 (as described in the experimental section below and in figure 8).

For all DNA constructs of the invention a preferred form is an expression vector. Thus, the DNA constructs allow reliable expression of the gene encoding a thermostable NAD-linked formate dehydrogenase in a thermophilic bacterium of the genus Bacillus transformed with the construct.

The DNA construct can be a plasmid. Preferably, the DNA construct can only replicate in the host thermophilic microorganism through recombination with the genome of the host thermophilic microorganism.

The DNA constructs described herein also preferably incorporate a suitable reporter gene as an indicator of successful transformation. The reporter gene can be an antibiotic resistance gene, such as a kanamycin or ampicillin resistance gene. Other reporters, such as green fluorescent protein (GFP) and beta-galactosidase (lacZ) may be utilised as appropriate. The DNA constructs may incorporate multiple reporter genes, as appropriate. Loss of reporter function is, in subsequent generations, indicative of integration of the gene encoding a thermostable NAD-linked formate dehydrogenase, together with appropriate flanking regions.

In a still further aspect, the invention relates to a thermophilic bacterium of the genus Bacillus comprising a DNA construct of the invention. Preferred recipient microorganisms are heteroloactate fermentative microorganism. In particular, the invention preferably relates to *Bacillus stearothermophilus.* The bacterium may be derived from strain LLD-R or LLD-15 for example. The thermophilic microorganism may be *Geobacillus thermoglucosidasius.*

Further disclosed herein is the use of a thermophilic bacterium of the genus Bacillus of the invention in fermentation, and in particular for the production of ethanol.

Similarly, the invention relates to a fermentation process for the production of ethanol comprising supplying a thermophilic thermophilic bacterium of the genus Bacillus of the invention with sugars. Microorganisms constructed according to the present invention are particularly suitable for high ethanol yield and volumetric productivity under optimal growth conditions. Accordingly, any microorganism of the invention may be used in any fermenter configuration, such as batch, fed-batch or continuous fermentation processes. The fermentation process can be a fed-batch process.

One of the principal benefits of using microorganisms such as thermophilic bacteria to produce bioethanol is that, unlike yeasts, they are capable of fermenting a wide range of sugars derived from agricultural waste products such as hemicelluloses. The sugars used in the fermentation processes of the invention may be derived from biomass. Fermentation can be of mixed sugars. The mixed sugars may include pentose sugars, preferably a majority of pentose sugars.

The fermentation process can be maintained in redox balance. This is particularly critical with thermophiles since, unlike mesophiles, sugar uptake appears to be unregulated in these microorganisms. Preferably, this is achieved through use of feedback sensors.

Whilst thermophilic bacteria have low tolerance to ethanol, this can conveniently be overcome in the fermentation processes of the invention by regular or continuous removal of ethanol. This ensures that the ethanol concentration in the fermentation is kept below the ethanol tolerance of the thermophilic bacteria of the invention. Ethanol may be continuously and conveniently removed from the high temperature fermentation by evaporation or distillation, such as membrane and/or mild vacuum evaporation for example.

The invention will now be described with reference to the following non-limiting description and figures.

### Brief description of the figures

Figure 1 shows the effect of various conditions on metabolic pathways in a thermophilic microorganism in which the lactate dehydrogenase pathway has been inactivated. The shade and thickness of the arrows indicate the relative dominance of the respective metabolic pathways.
   A. Metabolic pathways active at neutral pH and in the presence of low sugars. Here the pyruvate formate lyase pathway dominates.
   B. Metabolic pathways active at low pH and in the presence of low sugars. Here an anaerobic pyruvate dehydrogenase pathway dominates.
   C. Metabolic pathways active at low pH and in the presence of high sugars. Here the cells experience metabolic stress and fall out of redox balance leading to so called "redox death".
   D. Metabolic pathways active in thermophilic microorganisms of the present invention, at neutral pH and in the presence of high sugars. Here, the novel PFL-FDH pathway is dominant and ethanol and CO₂ are the only anaerobic products.
Figure 2 sets forth the nucleotide sequence of the synthetic *fdh* gene produced by codon optimisation to maximise thermostability. The DNA sequence of the *fdh* open reading frame is flanked by promoter and terminator (italics) regions. -35 and -10 boxes of the promoter are underlined. To clone the construct in a suitable vector, *Xba*1 sites were introduced on both sides of the sequence.
Figure 3 shows the nucleotide sequence of the Insertion Sequence (IS) of *B. stearothermophilus* Strain LLD-15. The 9 bp inverted repeat ends are shown in bold font.
Figure 4 is a schematic representation of the pCR-Blunt derivative plasmid pCR-F1. The plasmid includes a codon optimised *fdh1* gene under the control of the *ldh* promoter, cloned into pCR-Blunt at the unique *Xba*1 site.
Figure 5 is a schematic representation of the pUB110 derivative plasmid pUB-ISF1. The plasmid includes a codon optimised *fdh1* gene under the control of the *ldh* promoter, derived from the pCR-F1 plasmid and also an insertion sequence (IS) derived from the known Bacillus strain LLD-15.
Figure 6 is a schematic representation of the pUC18 derivative plasmid called pUCK. The plasmid includes a kanamycin resistance gene cloned from plasmid pUB110 into the unique *Zra*1 restriction sitein pUC18.
Figure 7 is a schematic representation of the pUCK derivative pUCK-LC. The plasmid carries an *ldh* gene with a deletion of 363 bp in the middle of the ORF.
Figure 8 is a schematic representation of the pUCK derivative pUCK-ldhB. The plasmid contains 750 bp of the *ldh* gene, including the downstream region of the gene.
Figure 9 is a schematic representation of the pUCK-LF1 plasmid. This plasmid is a pUCK derivative incorporating a gene integrating cassette containing the *fdh* gene under the control of the *ldh* promoter.

### Description of the Invention

### Materials

### Media and buffers

LB medium: Tryptone 10 g; Yeast Extract 5 g; NaCl 10 g; deionised water to 1 L
   Adjusted pH to 7 and autoclaved to sterilize
   For plate medium 20 g/l agar was added to the medium before autoclaving, cooled to 55°C and poured into sterile Petri dishes (approx. 20 ml/plate).
   For LB-amp plates filter-sterilised ampicillin solution was added to final concentration of 50 µg /ml before pouring the Petri plates.
SOC Medium: Tryptone 2.0 g; Yeast Extract 0.5 g; NaCl 0.05 g; MgCl₂.6H₂O 0.204 g;
   MgSO₄.7H₂O 0.247 g; Glucose 0.36 g; deionised H₂O to 100 ml. Dissolved, adjusted the pH to 7.0 and filter sterilised.
TGP Medium: Tryptone 17 g; Soya peptone 3 g; K₂HPO₄ 2.5 g; NaCl 5 g; Na pyruvate 4 g; glycerol 4 ml; deionised water to 1 L. Adjusted pH to 7 and autoclaved to sterilize.
   For plate medium, 20 g/l agar was added in the medium before autoclaving, cooled to 55°C and poured into sterile Petri dishes (approx. 25 ml/plate).
   For TGP-kan plates, filter-sterilised kanamycin solution to final concentration of 10 µg /ml was added before pouring the Petri plates.
TH buffer: Trehalose 272 mM; HEPES (pH 7.5 with KOH) 8 mM; double distilled H₂O to 1 L

### Microbial strains

E. coli DH5-alpha-Chemically competent cells were purchased from Invitrogen (Cat.18265-017).
   Bacillus subtilis subsp. Subtilis-German culture collection, DSMZ (DSM No. 10)
   Bacillus stearothermophilus. strain LLD-R - Deposited as NCIMB 12403
   Bacillus stearothermophilus. strain LLD-15 - Deposited as NCIMB 12428

### Plasmids

Plasmid pCR-Blunt and pCR-TOP02 were obtained from Invitrogen
Plasmid pUB110 - Bacillus subtilis BD170 strain harbouring this plasmid was obtained from the German culture collection, DSMZ (DSM No. 4514).
Plasmid pUC18 was obtained from Sigma-Aldrich.

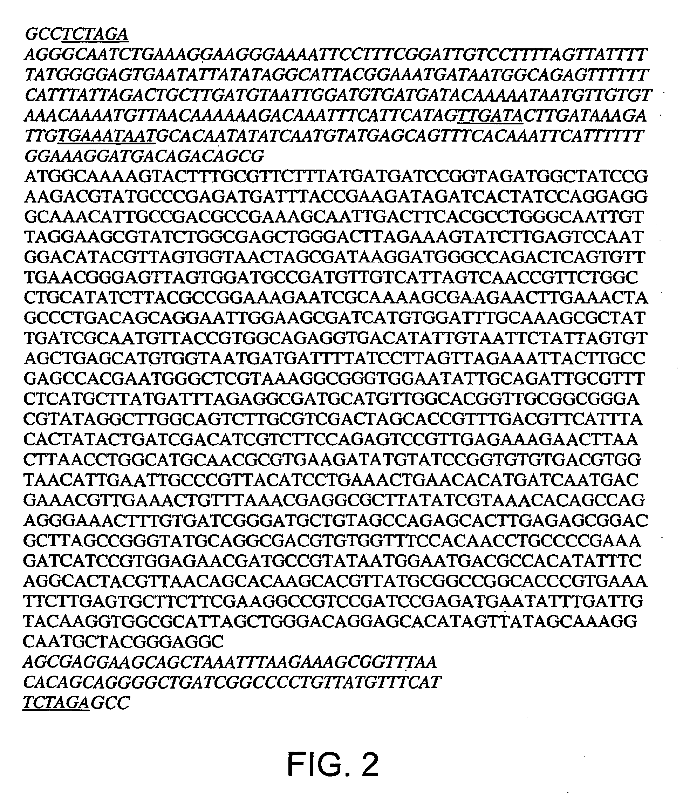

### Example 1. Construction of a synthetic formate dehydrogenase gene (Fig. 2)

An amino acid sequence (NCBI Protein Database Accession No P33160 - SEQ ID NO:3) of *Pseudomonas sp 101* formate dehydrogenases was back translated into DNA sequence with optimised codons for *Geobacillus thermoglucosidasius.* A promoter and a rho-independent terminator region from a Bacillus strain were added, upstream and downstream of the translated sequence respectively (Figure 2). The novel sequence showed less than 40% similarity with known fdh gene sequences (37% identity with known fdh1 gene). *Xba*1 sites were designed into both sides of the construct to facilitate its cloning into suitable vectors.

The desired sequence was synthesized using the method of Gao et al (see Xinxin Gao, Peggy Yo, Andrew Keith, Timothy J. Ragan and Thomas K. Harris (2003). Nucleic Acids Research, 31 (22), e143) and cloned into pCR-Blunt at its unique *Xba*1 position. The resulting vector pCR-F1 (Figure 4) was introduced into *E. coli* DH5 alpha cells and the positive clones were confirmed by PCR and restriction analysis.

Two alternative strategies are available to insert and express this synthetic *fdh* gene in the genome of target Bacilli as shown in the following examples.

### Example 2. Insertion of the fdh gene into multiple (IS) sites

This strategy applies to strains such as *Bacillus stearothermophilus* strain LLD-R that contain an Insertion Sequence (IS) that frequently recombines at multiple insertion sites. A vector carrying the *fdh* gene and this IS sequence is expected to integrate stably at one or more of such locations

### Construction of plasmid pUB-ISF1 (Figure 5)

Firstly, the known Insertion Sequence of strain LLD-R (SEQ ID NO: 5 and Figure 3) is PCR amplified using a forward primer (AGTACTGAAATCCGGATTTGATGGCG - SEQ ID NO:6) and a reverse primer (AGTACTGCTAAATTTCCAAGTAGC - SEQ ID NO:7) with *B. stearothermophilus* strain LLD-15 as the template. Sca1 restriction sites are introduced in the both ends of the sequence. The PCR product is first cloned in plasmid pCR-TOPO2.1 and the resulting plasmid pCR-IS is then introduced into E.coli DH5 alpha cells and used to isolate the IS region by *Sca*1 restriction digestion. The isolated IS is then cloned in pUB110 at its unique *Sca*1 site and the resulting plasmid pUB-IS is introduced into *Bacillus subtilis.*

Then a 1.5 kb fragment containing the *ldh* promoter and the *fdh* gene are digested from the pCR-F1 plasmid using *Xba*1 restriction enzyme, and cloned in plasmid pUB-IS that was already linearised with the same enzyme. The resulting plasmid pUB-ISF1 (Figure 5) is then introduced into *B. subtilis* and positive clones are selected on TGP-kan plates and confirmed by PCR and restriction analysis.

### Integration of the fdh gene into strain LLD-R

Plasmid pUB-ISF1 is then methylated in vitro with *Hae*III methylase enzyme and then *Bacillus stearothermophilus* strain LLD-R or its *ldh*-deleted strains (see Example 3) cells are transformed with the methylated pUB-ISF1 plasmid. Positive clones are selected after 48 hours on TGP-Kan plates at 50°C, and analysed by PCR amplification of the fdh gene.

The *fdh* gene is then integrated in the chromosome by growing a transformed clone in TGP-Kan medium at 60-65°C for a few generations and selecting on TGP-Kan plates. The positive clones are analysed for presence of the *fdh* gene and then screened for ethanol production and C5 (pentose) and C6 (hexose) sugar utilisation in shake flasks and in fermenters.

### Example 3. Construction of ldh-deleted strains.

The first step is to clone a Bacillus kanomycin resistance marker (kan) and a cassette carrying the ldh gene of *B. stearothermophilus* strain LLD-R into plasmid pUC18, which can replicate only in gram negative microorganisms.

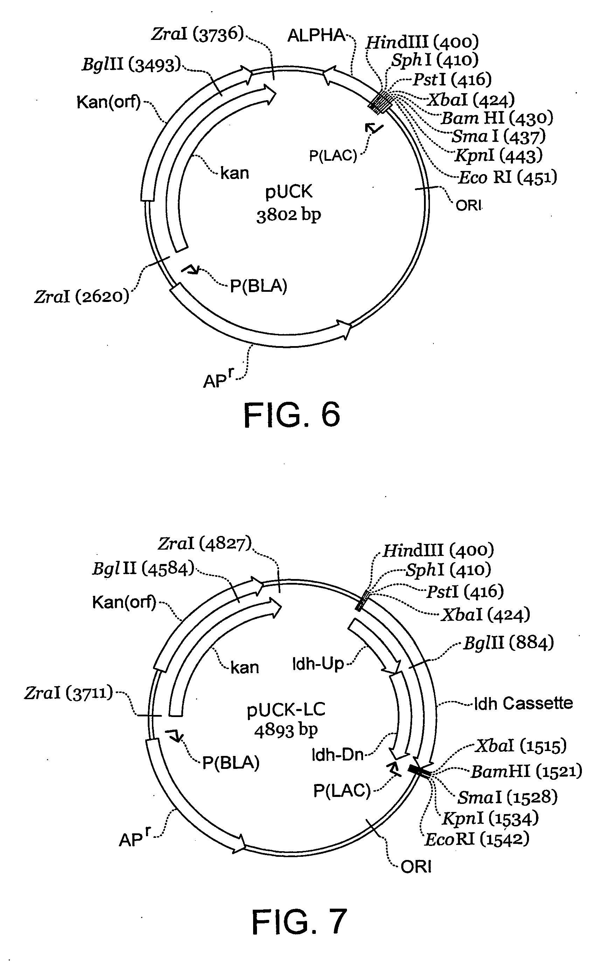

### Construction of a Bacillus cloning vector. Plasmid pUCK (Figure 6).

A kanamycin resistance gene (kan) was cloned in plasmid pUC18 at its unique *Zra*1 site which is outside of any coding region and of the reporter gene (lacZ) in the plasmid. To clone the kan gene, a 1.13 kb fragment containing the kanamycin resistance gene was PCR amplified with the primers:
kan-BsZ-F (ACACAGACGTCGGCGATTTGATTCATAC - SEQ ID NO:10) and
kan-BsZ-R (CGCCATGACGTCCATGATAATTACTAATACTAGG - SEQ ID NO:11)
using plasmid pUB110 as template. The *Zra*1 sites were introduced at both ends of the kan gene through the primers. The PCR product was then digested with *Zra*1 restriction endonuclease enzyme and ligated with previously *Zra*1-digested and dephosphorylated plasmid pUC18. The resulting plasmid pUCK (Figure 6) was then introduced into E. coli DH5 alpha cells. Positive clones were selected on LB-amp plates and confirmed by PCR and restriction analysis.

### Construction of plasmid pUCK-LC (Fig. 7) which carries a deleted ldh gene

A 1.36 kb *ldh* cassette was designed to contain the whole *ldh* gene of strain LLD-R from which 363 bp of its ORF was deleted plus its flanks. The cassette was constructed by PCR amplification of the upper and lower regions of the *ldh* gene using strain LLD-R as template. These regions were then ligated and cloned in plasmid pUCK. *Bgl*II sites were introduced into the inner primers. The upper region was PCR amplified using the following primers:
LC-U-F1 (AGGGCAATCTGAAAGGAAGGGAAAATTCC - SEQ ID NO:12) and
LC-UB-R1 TGCACAGATCTCCACCAAATCGGCGTC - SEQ ID NO:13).

The lower region was PCR amplified using the following primers:
LC-DB-F1 (TTGAGCAGATCTTGATGCAAAACGATAAC - SEQ ID NO:14) and
LC-D-R1 (TAAAGCCGATGAGCAGCAGTTGAAG - SEQ ID NO:15).

The PCR products were digested with BglII restriction endonuclease enzyme and ligated using T4 DNA ligase enzyme. Using the ligate as template, the ldh-cassette was then PCR amplified using as primers:
LC-UX-F2 (ATATTATCTAGACATTACGGAAATGATAATGGC - SEQ ID NO:16) and
LC-DX-R2 (TCACAATCTAGACAATCGGCCATAAAC - SEQ ID NO:17).

*Xba*I sites were introduced at the both ends of the cassette via the primers. The PCR product was then digested with *Xba*I enzyme and cloned into plasmid pUCK pre-digested with the same enzyme and dephosphorylated. The resulting plasmid pUCK-LC was then introduced into E. coli DH5 alpha. The positive clones were selected on LB-amp plates and confirmed by PCR and restriction analysis.

### Construction of ldh deleted strains

Plasmid pUCK-LC is methylated in vitro with *Hae*III methylase enzyme and wild type thermophile cells (e.g. strain LLD-R) are transformed into the methylated plasmid by electroporation (1000 V, 201 ohms, 25 micro-Faraday, and 5 milli-seconds). Positive clones are selected on TGP-Kan plates at 65°C and confirmed as single cross-over events by PCR amplification of the kan gene.

To achieve gene deletion by double cross-over, the positive clones are grown in TGP medium for a few generations (about 5 sub-cultures) and clones which can grow on TGP plates but not on TGP-kan plates are selected. The positive clones are then confirmed as *ldh* gene deletions and for the absence of the kanamycin gene by PCR analysis. The clones are then characterised for ethanol production and C5 and C6 sugar utilisation in shake flasks and in fermenters.

### Example 4. Simultaneous insertion of the fdh gene and deletion of the ldh gene.

This alternative strategy is broadly applicable to a wide class of heterolactate fermentative microorganisms as well as thermophilic Bacilli, though the latter will be used as illustration.

### Construction of plasmid pUCK-LF (Fig.9)

A gene integrating cassette containing the *fdh* gene plus the whole *ldh* gene and about 300 bp of upstream and downstream flanking regions is cloned into plasmid pUCK. In this construct, the first 450 bp of the ldh open reading frame are replaced with the *fdh* gene in such a way that the gene expression becomes under control of the *ldh* gene promoter.

To achieve this a DNA fragment of about 750 bp containing the downstream region of the *ldh* gene is PCR amplified using;
LDHB-X-F1 (GAACGATTCTAGATACAGCAAGATTCCGC - SEQ ID NO:8) and
LDHB-E-R1 (GTTTGCGAATTCATAGACGGACGCAG - SEQ ID NO:9) as primers and *Bacillus stearothermophilus* strain LLD-R as template. *Xba*1 and *EcoR*1 sites are thus introduced in the ends of the PCR fragment. The PCR fragment is then digested and directionally cloned in plasmid pUCK between the *Xba*1 and *EcoR*1 sites. The resulting plasmid, pUCK-ldhB (Figure 8) is introduced into *E. coli* DH5 alpha and positive clones are selected on LB-amp plates and confirmed by PCR and restriction

Then, a 1.5 kb fragment containing the *ldh* promoter and the *fdh* gene are digested out from the pCR-F1 plasmid using *Xba*1 restriction enzyme and cloned into plasmid pUCK-ldhB (Figure 8) which was already linearised with the same enzyme. The resulting plasmid pUCK-LF1 (Figure 9) is introduced into *E.coli* DH5 alpha cells and clones are selected on LB-Amp plates. Positive clones and the correct orientation of the construct are confirmed by PCR and restriction analysis.

### Construction of strains that make ethanol by the novel PFL-FDH pathway.

Plasmid pUCK-LF1 is methylated in vitro with *Hae*III methylase enzyme, and target wild type cells (e.g. strain LLD-R cells) are transformed with the methylated plasmid and selected on TGP-Kan plates at 60°C. The positive clones represent single cross-over events and are analysed by PCR amplification of the *fdh* gene.

To achieve double cross-over gene integration, clones that grow on TGP plates but not on TGP-Kan plates are selected. The positive clones are then confirmed for the presence of the *fdh* gene and absence of the kanamycin gene. Finally the clones are screened for ethanol production and C5 and C6 sugar utilisation in shake flasks and in fermenters.

## Claims

1. A thermophilic bacterium of the genus Bacillus lacking lactate dehydrogenase activity and which has pyruvate formate lyase activity, **characterised in that** the thermophilic bacterium contains a gene encoding an NAD-linked formate dehydrogenase.

2. The thermophilic bacterium of claim 1 wherein the gene encoding an NAD-linked formate dehydrogenase is integrated into the genome of the thermophilic bacterium.

3. The thermophilic bacterium of claim 1 or 2 wherein the gene encoding an NAD-linked formate dehydrogenase is expressed from its own promoter or from a promoter of the thermophilic bacterium.

4. The thermophilic bacterium of any one of claims 1 to 3 wherein the gene encoding an NAD-linked formate dehydrogenase is inserted into the lactate dehydrogenase gene of the thermophilic bacterium, thus inactivating the lactate dehydrogenase activity of the thermophilic bacterium.

5. The thermophilic bacterium of any preceding claim wherein the gene encoding an NAD-linked formate dehydrogenase comprises the nucleotide sequence set forth as SEQ ID NO: 1 or 2.

6. The thermophilic bacterium of any preceding claim which has been transformed with a DNA construct comprising a gene encoding an NAD-linked formate dehydrogenase operably linked to an upstream region of a gene encoding a lactate dehydrogenase wherein the upstream region includes the promoter and further comprising at least part of the lactate dehydrogenase gene downstream of the gene encoding an NAD-linked formate dehydrogenase such that the gene encoding an NAD-linked formate dehydrogenase is interposed between a sufficient portion of the lactate dehydrogenase gene on either side to facilitate integration of the gene encoding an NAD-linked formate dehydrogenase by recombination with a lactate dehydrogenase gene in the genome of the thermophilic bacterium.

7. A gene encoding a thermostable NAD-linked formate dehydrogenase comprising the nucleotide sequence set forth as SEQ ID NO:1.

8. A DNA construct comprising a regulatory sequence operably linked to a gene encoding a thermostable NAD-linked formate dehydrogenase comprising the nucleotide sequence set forth as SEQ ID NO:1.

9. A DNA construct comprising a gene encoding an NAD-linked formate dehydrogenase, optionally a thermostable NAD-linked formate dehydrogenase, and a transposable DNA element capable of integration into the genome of a thermophilic bacterium of the genus Bacillus which facilitates integration of the gene encoding an NAD-linked formate dehydrogenase into the genome of the thermophilic bacterium transformed with the DNA construct.

10. A DNA construct comprising a gene encoding an NAD-linked formate dehydrogenase, optionally a thermostable NAD-linked formate dehydrogenase, operably linked to an upstream region of a gene encoding a lactate dehydrogenase, wherein the upstream region includes the promoter.

11. A thermophilic bacterium of the genus Bacillus comprising the DNA construct as defined in any one of claims 8 to 10.

12. Use of a thermophilic bacterium as claimed in any one of claims 1 to 6 or as claimed in claim 11 for the production of ethanol.

13. A fermentation process for production of ethanol comprising supplying a thermophilic bacterium as claimed in any one of claims 1 to 6 or as claimed in claim 11 with sugars.

## Patentansprüche

1. Thermophiles Bakterium der Gattung Bacillus, dem die Lactatdehydrogenase-Aktivität fehlt und welches Formiatacetyltransferase-Aktivität aufweist, **dadurch gekennzeichnet, dass** das thermophile Bakterium ein Gen enthält, das eine NAD-gekoppelte Formiatdehydrogenase kodiert.

2. Thermophiles Bakterium nach Anspruch 1, wobei das Gen, das eine NAD-gekoppelte Formiatdehydrogenase kodiert, in das Genom des thermophilen Bakteriums integriert ist.

3. Thermophiles Bakterium nach Anspruch 1 oder 2, wobei das Gen, das eine NAD-gekoppelte Formiatdehydrogenase kodiert, von seinem eigenen Promotor oder von einem Promotor des thermophilen Bakteriums exprimiert wird.

4. Thermophiles Bakterium nach einem der Ansprüche 1 bis 3, wobei das Gen, das eine NAD-gekoppelte Formiatdehydrogenase kodiert, in das Lactatdehydrogenase-Gen des thermophilen Bakteriums eingefügt ist, wodurch die Lactatdehydrogenase-Aktivität des thermophilen Bakteriums inaktiviert wird.

5. Thermophiles Bakterium nach einem der vorhergehenden Ansprüche, wobei das Gen, das eine NAD-gekoppelte Formiatdehydrogenase kodiert, die als SEQ ID NO: 1 oder 2 dargelegte Nucleotidsequenz umfasst.

6. Thermophiles Bakterium nach einem der vorhergehenden Ansprüche, welches mit einem DNA-Konstrukt, umfassend ein Gen, das eine NAD-gekoppelte Formiatdehydrogenase kodiert, operativ verknüpft mit einer stromaufwärts liegenden Region eines Gens, das eine Lactatdehydrogenase kodiert, wobei die stromaufwärts liegende Region den Promotor umfasst, und weiterhin umfassend mindestens einen Teil des Lactatdehydrogenase-Gens stromabwärts von dem Gen, das eine NAD-gekoppelte Formiatdehydrogenase kodiert, so dass das Gen, das eine NAD-gekoppelte Formiatdehydrogenase kodiert, zwischen einem hinreichenden Anteil des Lactatdehydrogenase-Gens an beiden Seiten eingefügt ist, um die Integration des Gens, das eine NAD-gekoppelte Formiatdehydrogenase kodiert, durch Rekombination mit einem Lactatdehydrogenase-Gen in dem Genom des thermophilen Bakteriums zu erleichtern, transformiert würde.

7. Gen, das eine thermostabile NAD-gekoppelte Formiatdehydrogenase kodiert, umfassend die als SEQ ID NO: 1 dargelegte Nucleotidsequenz.

8. DNA-Konstrukt, umfassend eine regulatorische Sequenz, die mit einem Gen, das eine thermostabile NAD-gekoppelte Formiatdehydrogenase kodiert, umfassend die als SEQ ID NO: 1 dargelegte Nucleotidsequenz, operativ verknüpft ist.

9. DNA-Konstrukt, umfassend ein Gen, das eine NAD-gekoppelte Formiatdehydrogenase, gegebenenfalls eine thermostabile NAD-gekoppelte Formiatdehydrogenase, kodiert und ein transponierbares DNA-Element, das zur Integration in das Genom eines thermophilen Bakteriums der Gattung Bacillus fähig ist, das die integration des Gens, das eine NAD-gekoppelte Formiatdehydrogenase kodiert, in das mit dem DNA-Konstrukt transformierte Genom des thermophilen Bakteriums erleichtert.

10. DNA-Konstrukt, umfassend ein Gen, das eine NAD-gekoppelte Formiatdehydrogenase, gegebenenfalls eine thermostabile NAD-gekoppelte Formiatdehydrogenase, kodiert, operativ verknüpft mit einer stromaufwärts liegenden Region eines Gens, das eine Lactatdehydrogenase kodiert, wobei die stromaufwärts liegende Region den Promotor umfasst.

11. Thermophiles Bakterium der Gattung Bacillus, umfassend das DNA-Konstrukt, wie in einem der Ansprüche 8 bis 10 definiert.

12. Verwendung eines thermophilen Bakterium, die in einen der Ansprüche 1 bis 6 oder wie in Anspruch 11 beansprucht, zur Produktion von Ethanol.

13. Fermentationsverfahren zur Production von Ethanol, umfassend die Versorgung eines thermophilen Bakteriums, wie in einem der Ansprüche 1 bis 6 oder wie in Anspruch 11 beansprucht, mit Zuckern.

## Revendications

1. Bactérie thermophile du genre Bacillus dépourvue d'activité de lactate-déshydrogénase et qui a une activité de pyruvate-formiate-lyase, ladite bactérie thermophile étant **caractérisée en ce qu'**elle contient un gène codant pour une formiate-déshydrogénase liée au NAD.

2. Bactérie thermophile suivant la revendication 1, dans laquelle le gène codant pour une formiate-déshydrogénase liée au NAD est intégré au génome de la bactérie thermophile.

3. Bactérie thermophile suivant la revendication 1 ou 2, dans laquelle le gène codant pour une formiate-déshydrogénase liée au NAD est exprimé à partir de son propre promoteur ou à partir d'un promoteur de la bactérie thermophile.

4. Bactérie thermophile suivant l'une quelconque des revendications 1 à 3, dans laquelle le gène codant pour une formiate-déshydrogénase liée au NAD est inséré dans le gène de lactate-déshydrogénase de la bactérie thermophile, ce qui inactive l'activité de lactase-déshydrogénase de la bactérie thermophile.

5. Bactérie thermophile suivant l'une quelconque des revendications précédentes, dans laquelle le gène codant pour une formiate-déshydrogénase liée au NAD comprend la séquence de nucléotides indiquée en tant que SEQ ID N° 1 ou 2.

6. Bactérie thermophile suivant l'une quelconque des revendications précédentes, qui a été transformée avec un produit d'assemblage d'ADN comprenant un gène codant pour une formiate-déshydrogénase liée au NAD liée de manière fonctionnelle à une région amont d'un gène codant pour une lactate-déshydrogénase, dans laquelle la région amont comprend le promoteur, et comprenant en outre au moins une partie du gène de lactate-déshydrogénase en aval du gène codant pour une formiate-déshydrogénase liée au NAD de telle sorte que le gène codant pour une formiate-déshydrogénase liée au NAD soit interposé entre une portion suffisante du gène de lactate-déshydrogénase de chaque côté pour faciliter l'intégration du gène codant pour une formiate-déshydrogénase liée au NAD par recombinaison avec un gène de lactate-déshydrogénase dans le génome de la bactérie thermophile.

7. Gène codant pour une formiate-déshydrogénase liée au NAD thermostable, comprenant la séquence de nucléotides indiquée en tant que SEQ ID N° 1.

8. Produit d'assemblage d'ADN comprenant une séquence régulatrice liée de manière fonctionnelle à un gène codant pour une formiate-déshydrogénase liée au NAD thermostable comprenant la séquence de nucléotides indiquée en tant que SEQ ID N° 1.

9. Produit d'assemblage d'ADN comprenant un gène codant pour une formiate-déshydrogénase liée au NAD, facultativement une formiate-déshydrogénase liée au NAD thermostable, et un élément d'ADN transposable apte à l'intégration dans le gène d'une bactérie thermophile du genre Bacillus qui facilite l'intégration du gène codant pour une formiate-déshydrogénase liée au NAD dans le génome de la bactérie thermophile transformée avec le produit d'assemblage d'ADN.

10. Produit d'assemblage d'ADN comprenant un gène codant pour une formiate-déshydrogénase liée au NAD, facultativement une formiate-déshydrogénase liée au NAD thermostable, lié de manière fonctionnelle à une région amont d'un gène codant pour une lactate-déshydrogénase, dans lequel la région amont comprend le promoteur.

11. Bactérie thermophile du genre Bacillus comprenant le produit d'assemblage d'ADN tel que défini dans l'une quelconque des revendications 8 à 10.

12. Utilisation d'une bactérie thermophile suivant l'une quelconque des revendications 1 à 6 ou suivant la revendication 11, pour la production d'éthanol.

13. Procédé de fermentation pour la production d'éthanol, comprenant la fourniture de sucres à une bactérie thermophile suivant l'une quelconque des revendications 1 à 6 ou suivant la revendication 11.
